# EUROPEAN PATENT SPECIFICATION

(11) **EP 2 523 632 B1**
(45) Date of publication and mention of the grant of the patent: **23.11.2016**
(21) Application number: 11700596.7
(22) Date of filing: 11.01.2011
(51) Int. Cl.: A61F 2/16, A61F 9/007, A61F 2/00

(54) **FIXATION OF OPTHALMIC IMPLANTS**
FIXIERUNG VON AUGENIMPLANTATEN
FIXATION D'IMPLANTS OPHTALMIQUES

(30) Priority: 11.01.2010 US 685426
(43) Date of publication of application: 21.11.2012
(73) Proprietor: Abbott Medical Optics Inc., Santa Ana, CA 92705 (US)
(72) Inventor: REISIN, Carina, R., Tustin CA 92782 (US); KADZIAUSKAS, Kenneth, E., Coto de Caza CA 92679 (US); BASINGER, Brooke, C., Long Beach CA 90802 (US); BUMBALOUGH, Timothy, R., Fullerton CA 92832 (US)
(74) Representative: Hoffmann Eitle
(86) International application number: PCT/US2011/020778
(87) International publication number: WO 2011/085349

(56) References cited:
- WO-A1-2008/108524
- WO-A1-2011/031557
- WO-A1-2011/031557
- US-A- 4 946 436
- US-A- 5 002 571
- US-A- 5 882 327
- US-A1- 2008 140 192

## Description

### Field of the Invention

The present disclosure relates to ophthalmic implants and related methods, and more particularly to intraocular lenses and other ophthalmic implants (e.g., glaucoma shunts) with improved fixation and/or control of cellular growth.

The present invention relates to an accommodating intraocular lens as defined in claim 1.

### Background of the Invention

A human eye can suffer diseases that impair a patient's vision. For instance, a cataract may increase the opacity of the lens, causing blindness. To restore the patient's vision, the diseased lens may be surgically removed and replaced with an artificial lens, known as an intraocular lens, or IOL. In other cases, glaucoma may result in a gradual and undesirable increase of intraocular pressure (IOP). In such instances, a shunt may be implanted to help control pressure within the eye. In either case, it is generally desirable to maintain the ocular device at a fixed location within the eye.

The simplest IOLs are monofocal IOLs that are fixed within the eye and have a single focal length or optical power. Unlike the eye's natural lens, which can adjust its focal length within a particular range in a process known as accommodation, these IOLs cannot generally accommodate. As a result, objects at a particular position away from the eye appear in focus, while objects at increasing distances away from that position appear increasingly blurred.

Bifocal or multifocal IOLs, which are also generally fixed within the eye, produce two or more foci in order to simulate the accommodation produced by the eye's natural lens. For example, one of the foci may be selected to provide distant vision, while a second focus is selected to provide near vision. While multifocal IOLs improve the ability of a subject to focus on objects over a range of distances, the presence of more than one focus generally results in reduced contrast sensitivity compared to monofocal IOLs. A multifocal IOL may also be used for presbyopic lens exchange. Presbyopia is the condition where the eye exhibits a progressively diminished ability to focus on objects over a range of distances. It is caused by a gradual loss of "accommodation" in the natural lens inside the eye due to age-related changes that make the lens harder and less elastic with the years.

An improvement over the fixed IOLs (either monofocal or multifocal) is an accommodating IOL, or AIOL, which can adjust its power and/or axial position within a particular range. As a result, the patient can clearly focus on objects over a range of distances from the eye in a way that is similar to that provided by the natural lens. This ability to accommodate may be of tremendous benefit for the patient, and more closely approximates the patient's natural vision than monofocal or multifocal IOLs. Such artificial implantable lenses can take the form of injectable IOLs (polymer material injected into the capsular bag), Deformable IOLs (the lens' optic shape change creates optical power change), axially moving IOLs, Dual Optics IOLs, etc, or some combination thereof. Alignment of AIOLs within the eye may be particularly important. Thus, reliable attachment means may be especially useful in assuring quality optical performance for AIOLs.

The human eye contains a structure known as the capsular bag, which surrounds the natural lens. The capsular bag is transparent, and serves to hold the lens. In the natural eye, accommodation is initiated in part by the ciliary muscle and a series of zonular fibers, also known as zonules. The zonules are located in a relatively thick band mostly around the equator of the lens, and impart a largely radial force to the capsular bag that can alter the shape and/or the location of the natural lens and thereby change its effective power and/or focal distance.

In a typical surgery in which the natural lens is removed from the eye, the lens material is typically broken up and vacuumed out of the eye, but the capsular bag is left generally intact. The remaining capsular bag is extremely useful in that it may be used to house an AIOL, which is acted on by the zonules to change shape and/or shift in some manner to affect the lens power and/or the axial location of the image.

The AIOL has an optic, which refracts light that passes through it and forms an image on the retina, and may also include a haptic, which mechanically couples the optic to the capsular bag or holds the AIOL in contact with the capsular bag. During accommodation, the zonules exert a force on the capsular bag, which in turn exerts a force on the optic. The force may be transmitted from the capsular bag directly to the optic or from the capsular bag through a haptic to the optic. In either case, the lens changes shape and/or position dynamically to keep an object in focus on the retina as its distance from the eye varies.

Desirably, the design of the AIOLs effectively translates the ocular forces of the natural accommodative mechanism of the eye [ciliary muscle - zonules - capsular bag] to maximize accommodation amplitude or range. Also, AIOLs may take into account the problem of lens epithelial cell (LECs) proliferation which can cause opacification and stiffening of the capsular bag over time. This phenomenon is caused by the wound healing reactions of the natural lens epithelial cells that remain on the inside of the capsular bag, often in the narrow ring around the equatorial region. Several methods to prevent the LECs from proliferating have been tried, including removing the LECs as much as possible, mechanically as well as pharmaceutically. Alternatively, design features such as square edge and spacers have been incorporated into the AIOLs.

As mentioned above, ocular implants may also be used in long-term glaucoma treatment. Glaucoma is a progressive disease of the eye characterized by a gradual increase of intraocular pressure (IOP). This increase in pressure is most commonly caused by stenosis or blockage of the aqueous outflow channel, resulting in excessive buildup of aqueous fluid within the eye. The implant solution typically involves suturing a small plate to the sclera in the anterior segment of the eye at the limbus, and inserting a drainage tube into the anterior chamber of the eye. Once implanted, the body forms scar tissue around the plate. Aqueous humor flow through the tube causes the tissues above the plate to lift and form a bleb. A bleb is a fluid filled space surrounded by scar tissue, somewhat akin to a blister. The fluid within the bleb then flows through the scar tissue at a rate which desirably regulates IOP. More recently, U.S. Patent Nos. 5,476,445 and 6,050,970 to Dr. George Baerveldt, et al. disclose glaucoma implants or shunts featuring a flexible plate that attaches to the sclera and a drainage tube positioned for insertion into the anterior chamber of the eye. This type of shunt is sold under the trade name Baerveldt® BG Series of glaucoma implants by Advanced Medical Optics (AMO) of Santa Ana, CA. The Baerveldt® device has an open tube without flow restricting elements. Temporary sutures are used to restrict fluid flow for a predetermined period, after which the bleb forms and fluid drainage is properly regulated. The temporary sutures are either biodegradable or removed in a separate procedure. This method works well, but the timing of suture dissolution is necessarily inexact, and a second procedure undesirable.

WO 2008/108524 A1 discloses an intraocular lens that is inserted inwardly into a capsular sac. An intraocular lens is inserted inwardly into a capsular sac including an optic portion and a haptic portion including a connection bar coupled to the optic portion and first support bar coupled to a circumference of the connection bar to be in contact with an inner surface of the capsular sac.

WO 2011/031557 A1, which was published after the filing date of the present application, discloses an accommodating intraocular lens with an optic (52) that changes shape in response to an ocular force exerted by the zonules of the eye. A haptic supports the optic around its equator and couples the optic to the capsular bag of the eye. A surface adherent improves the accommodative performance of the haptic, such that compressive/tensile forces may be more efficiently transferred from the haptic to optic. As one way to enhance force transfer, it is disclosed to provide a surface layer of an adhesive to the haptic and/or optic, for instance a reversible bioadhesive material. As a second way, it is disclosed that portions of the exterior surface of the IOL may have microfibers thereon that mimic the adhesive properties of Gecko feet.

In these and other situations, ophthalmic lenses, and related methods of fabrication and implantation of such lenses, are needed for securely attaching such implants in an eye of a human or animal subject. In some instances, reversal of the attachment means is desirable, for example, to allow the device to be more readily explanted or positioned during implantation. In addition, there exists a need for an AIOL with increased efficiency in converting an ocular force to a change in power and/or a change in axial location of the image, generally in a way which also reduces the problem of lens epithelial cell proliferation. There is also a need for an alternative to suturing glaucoma shunts in place.

Where in the following the word invention is used and/or features are presented as optional, this should be interpreted in such a way that protection is sought for the invention as claimed.

### Brief Description of the Drawings

Features and advantages of the present invention will become appreciated as the same become better understood with reference to the specification, claims, and appended drawings wherein:
Figure 1 is a vertical sectional view of a human eye.
Figure 2A is a vertical sectional view of a portion of an eye having an implanted intraocular lens, in an accommodative or "near" state.
Figure 2B is a vertical sectional view of the eye of Figure 2A, in a disaccommodative or "far" state for providing distant vision.
Figure 3 is a perspective view of an intraocular lens having a pair of axially spaced-apart and centered optics, and a plurality of convex haptic legs connecting the optics and radiating outward therefrom;
Figure 4 is an elevational view of the intraocular lens of Figure 3;
Figure 5 is a sectional view of the intraocular lens of Figure 3;
Figure 6A and 6B are vertical sectional views through an eye showing the implanted exemplary AIOL of Figures 3-5 in two states of accommodation;
Figure 7 is a perspective view of an intraocular lens having an optic within which is embedded a portion of an accommodative haptic, the accommodative haptic including a central vaulted portion, a plurality of spokes each having a unitary outer end, axially spaced apart bifurcated inner ends connected in two axially spaced planes, and central throughholes in the central vaulted portion;
Figure 8A is a vertical sectional view through an eye showing preparation of the inner surface of the capsular bag by application of a bio-adhesive;
Figure 8B is a vertical sectional view through an eye showing introduction of an injectable polymer AIOL into the capsular bag prepared as in Figure 8A;
Figure 9 is a perspective view of an exemplary glaucoma shunt that may be fixed in place using the principles described herein; and
Figure 10 is a bottom plan view of the glaucoma shunt of Figure 9 showing an exemplary distribution of an adhering surface.
Figure 11 is a perspective view of an accommodating intraocular lens according to an embodiment of the present invention, which shows an optic body and a support structure.
Figure 12 is a perspective view of the support structure of the accommodating intraocular lens shown in Figure 11.
Figure 13 is a plan view of the support structure shown in Figure 12.
Figure 14 is a cross sectional view of the accommodating intraocular lens shown in Figure 11.
Figure 15 is a magnified view sectional view of the accommodating intraocular lens shown in Figure 11.
Figure 16 is a flow chart outlining a method of implantation according to an embodiment of the present invention.

### Detailed Description of the Drawings

Embodiments of the present invention are generally directed to devices, substances, and methods for attaching ophthalmic devices, such as ophthalmic lenses, and/or controlling cellular growth after implantation of an ocular device. Embodiments of the present invention are particularly useful when used in conjunction with IOLs; however, may be used with a variety of ophthalmic devices, for example, with a shunt that is implanted to help control pressure within the eye. Embodiments of the present invention may provide immediate and/or reversible adhesion or adherence of an ophthalmic device within the capsular bag of an animal or human subject. Surface adherents or adhesives according to embodiments of the present invention are generally reversible, thus allowing an IOL to be explanted or readjusted subsequent to initial attachment within the eye. While potentially applicable to a variety of ophthalmic devices and IOLs, surface adherents or adhesives according to embodiments of the present invention may find particular use with accommodating IOLs, which may have attachment and alignment requirements that are especially critical.

In a healthy human eye, the natural lens is housed in a structure known as the capsular bag. The capsular bag is driven by a ciliary muscle and zonular fibers (also known as zonules) in the eye, which can alternately pull on or release on the capsular bag to change its shape. The motions of the capsular bag change the shape of the natural lens in order to change its power and/or the location of the lens, so that the eye can focus on objects at varying distances away from the eye in a process known as accommodation.

For some people suffering from cataracts, the natural lens of the eye becomes clouded or opaque. If left untreated, the vision of the eye becomes degraded and blindness can occur in the eye. A standard treatment is surgery, during which the natural lens is broken up, removed, and replaced with a manufactured intraocular lens. Typically, the capsular bag is left intact in the eye, so that it may house the implanted intraocular lens.

Because the capsular bag is capable of shape change, initiated by the capsular bag resiliency, ciliary muscle, and/or zonules, it is desirable that the implanted intraocular lens be configured to utilize the ocular forces produced thereby to change its power and/or location in the eye in a manner similar to that of the natural lens. Such an accommodating lens may produce improved vision over conventional monofocal or multifocal IOLs.

A desirable optic or optic body for an accommodating IOL is one that changes shape in response to an ocular force, for example, a squeezing or expanding radial force applied largely to the equator of the optic (e.g., by pushing or pulling on or near the edge of the optic, circumferentially around the optic axis). Under the influence of an ocular force, the optic of the IOL may bulge slightly in the axial direction, producing more steeply curved anterior and/or posterior faces, and producing an increase in the power of the optic. Likewise, an expanding radial force produces a decrease in the optic power by flattening the optic. This change in power is accomplished in a manner similar to that of the natural eye and is well adapted to accommodation.

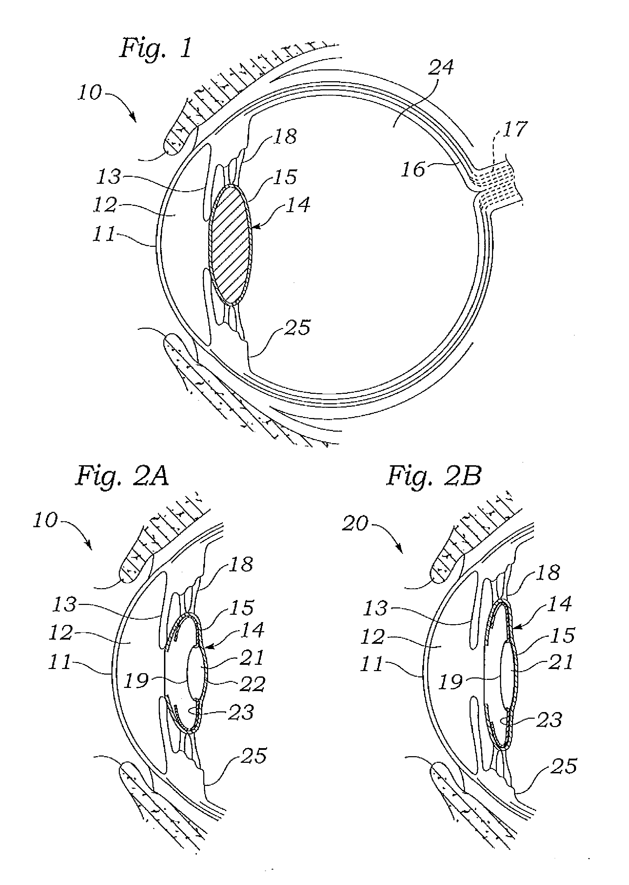

Figure 1 shows a human eye 10 in vertical section. Light enters from the left of Figure 1, and passes through the cornea 11, the anterior chamber 12, the iris 13, and enters the capsular bag 14. Prior to surgery, the natural lens occupies essentially the entire interior of the capsular bag 14. After surgery, the capsular bag 14 houses the intraocular lens. The intraocular lens is described in more detail below. After passing through the natural lens, light exits the posterior wall 15 of the capsular bag 14, passes through the posterior chamber 24, and is focused onto the retina 16, which detects the light and converts it to a signal transmitted through the optic nerve 17 to the brain.

Figure 2A shows the eye 10 in an accommodative state (e.g., for providing near vision) after an accommodating intraocular lens has been implanted. Figure 2B shows same accommodating intraocular lens when the eye is in a disaccommodative state for providing distant vision. A well-corrected eye forms an image at the retina 16. If the lens system (cornea + IOL) has too much or too little power, the image shifts axially along the optical axis away from the retina. The power required to focus on a close or near object is more than the power required to focus on a distant or far object. The difference between the "near" and "distant" powers is known typically as the add power or as the range of accommodation or accommodative range. A normal range of accommodation is about 2 to 4 diopters, which is considered sufficient for most patients, but some have a range of about 1 to 8 diopters. As used herein, the term "about" means within plus or minus 0.25 Diopters, when used in reference to an optical power.

The capsular bag is acted upon by the ciliary muscle 25 via the zonules 18, which change the shape of the capsular bag 14 by releasing or stretching it radially in a relatively thick band about its equator. Experimentally, it is found that the ciliary muscle 25 and/or the zonules 18 typically exert a total ocular force of up to about 10 grams of force, which is distributed generally uniformly around the equator of the capsular bag 14. As used herein, the term "about" means within plus or minus 0.5 grams of force, when used in reference to an ocular force. As used herein, an "ocular force" is a force produced by a human or animal eye to provide accommodation, for example, a force produce by the ciliary muscle, zonules, and/or capsular bag of an eye. In human eyes, an ocular force is generally be considered to be a force that is in a range from 0.5 gram force to 20 grams force, 0.5 gram force to 10 grams force, or 0.5 gram force to 6 grams force, where 1 gram force is equal to about 0.0098 Newtons. Although the range of ocular force may vary from one subject to another, it is noted that for each patient, the range of accommodation is limited by the total ocular force that can be exerted. It may be desirable that the intraocular lens be configured to vary its power over the full range of accommodation, in response to this limited range of ocular forces. In other words, it is desirable to have a relatively large change in power for a relatively small driving force. As used herein, the term "full range of accommodation" means a variation in optical power of an optic, lens, or lens system that is able to provide both distant and near vision, for example, a change in optical power of at least 3 Diopters or at least 4 Diopters.

The intraocular lens itself generally has two components, an optic 21, which is made of a transparent, deformable and/or elastic material, and a haptic or support structure 23, which holds the optic 21 in place and mechanically transfers forces on the capsular bag 14 to the optic 21. The haptic 23 may have an engagement member with a central recess that is sized to receive the peripheral edge of the optic 21. The haptic and optic may be refractive index matched to reduce unwanted reflections.

The lens desirably has a surface adherent or adhesive thereon, either on just the haptic 23 or also on the optic 21. Various surface adherents or adhesives are described herein, and any combination and placement of such adherents may be applied to the lens in Figures 2A and 2B to facilitate accommodation, as will be described. In general, under typical ocular forces, the level of adhesion is sufficient to prevent separation under between capsular bag 14 and areas of the support structure 23 containing an adherent or adhesive. In some embodiments, the adhesion over these areas of support structure 23 is at least 1 gram force per square centimeter, 10 grams force per square centimeter, or at least 100 grams force per square centimeter. In some embodiments, the adhesion is about 1 Newton per square centimeter, which value is typical for gecko feet microfibers.

When the eye 10 focuses on a relatively close object, as shown in Figure 2A, the zonules 18 relax and permit the capsular bag 14 to return to its natural shape in which it is relatively thick at its center and has more steeply curved sides. As a result of this action, the power of the lens increases (i.e., one or both of the radii of curvature can decrease, and/or the lens can become thicker, and/or the lens may also move axially), placing the image of the relatively close object at the retina 16. Note that if the lens could not accommodate, the image of the relatively close object would be located behind the retina, and would appear blurred.

Figure 2B shows a portion of an eye 20 that is focused on a relatively distant object. The cornea 11 and anterior chamber 12 are typically unaffected by accommodation, and are substantially identical to the corresponding elements in Figure 2A. To focus on the distant object, the ciliary muscle 25 contracts and the zonules 18 retract and change the shape of the capsular bag 14, which becomes thinner at its center and has less steeply curved sides. This reduces the lens power by flattening (i.e., lengthening radii of curvature and/or thinning) the lens, placing the image of the relatively distant object at the retina (not shown).

For both the "near" case of Figure 2A and the "far" case of Figure 2B, the intraocular lens itself changes shape in response to ocular forces provided by the ciliary muscles and/or the capsular bag. For a "near" object, the haptic 23 compresses the optic 21 at its edge, increasing the thickness of the optic 21 at its center and increasing the curvature of at least a portion of its anterior face 19 and/or its posterior face 15. As a result, the power of the optic 21 increases. For the "far" object, the haptic 30 expands, pulling on the optic 21 at its edge, and thereby decreasing the thickness of the optic 21 at its center and decreasing the curvature of at least a portion of its anterior face 19 and/or its posterior face 15. As a result, the lens power decreases.

For the "near" case shown in Figure 2A, the eye provides near vision, while for the "far" case shown in Figure 2B, the eye provides distant vision. As used herein, the term "near vision" means vision provided by an ophthalmic lens when placed within an eye of a subject, wherein a best optical performance or visual acuity occurs for objects located within a range of 25 cm to 40 cm from the subject, or at a distance at which the subject would generally place printed material for the purpose of reading. The distance range of 25 cm to 40 cm corresponds to a spectacle add power, or an accommodative power, of 4 Diopters to 2.5 Diopters, respectively. As used herein, the term "intermediate vision" means vision provided by an ophthalmic lens when placed within the eye, wherein a best optical performance or visual acuity occurs for objects located within a range of 40 cm (an accommodative or spectacle add power of 2.5 Diopters) to 2 meters (an accommodative or spectacle add power of 0.5 Diopters) from the subject. As used herein, the term "distant vision" means vision provided by an ophthalmic lens when placed within the eye, wherein a best optical performance or visual acuity occurs for objects located at a distance of 6 meters or greater from the subject. As used herein, an intraocular lens add power, or intraocular lens accommodative power, is equal to a corresponding spectacle add power multiplied by 0.8.

Unless otherwise specified, the power of an IOL is the optical power or effective optical power when the IOL or corresponding support or haptic structure is in a natural, relaxed, or unstressed state. As used herein a "natural state", "relaxed state", or "unstressed state" means a state of an IOL, optic, or corresponding haptic or support structure in which no external forces other than gravity are acting on the IOL, optic, or corresponding haptic or support structure. The power of an IOL may be selected such that the IOL has an accommodative bias or a disaccommodative bias. As used herein, an IOL has a "disaccommodative bias" when the IOL has an optical power suitable for providing distant vision to a subject eye when the IOL is in a natural or unstressed state. As used herein, an IOL has an "accommodative bias" when the IOL has an optical power suitable for providing near vision to a subject eye when the IOL is in a natural or unstressed state. As used herein, an IOL has an "intermediate bias" when the IOL has an optical power suitable for providing intermediate vision to a subject eye when the IOL is in a natural or unstressed state.

Note that the specific degrees of change in curvature of the anterior and posterior faces may depend on the nominal curvatures. Although the optic 21 is drawn as bi-convex, it may also be plano-convex, meniscus or other lens shapes. In all of these cases, the optic is compressed or stretched by forces applied by the haptic to the edge and/or faces of the optic. In addition, there may be some axial movement of the optic. In some embodiments, the haptic is configured to transfer the generally symmetric radial forces symmetrically to the optic to change the shape or surface curvature of the optic in an axisymmetric way. However, in alternate embodiments the haptic is configured non-uniformly (e.g., having different material properties, thickness, dimensions, spacing, angles or curvatures), to allow for non-uniform transfer of forces by the haptic to the optic. For example, this could be used to combat astigmatism, coma or other asymmetric aberrations of the eye/lens system. The optic may optionally have one or more diffractive elements, one or more multifocal elements, and/or one or more aspheric elements.

Certain exemplary embodiments herein provide a haptic partly embedded within an adjustable or accommodative central optic. The haptic transmits forces to alter at least one of the shape and the thickness of the adjustable optic. The materials of the haptic and optic may have similar compressive or spring moduli, to encourage direct transfer of forces and reduce uneven expansion/contraction and accompanying tension therebetween, though the haptics are generally somewhat stiffer to be capable of transmitting capsular forces. Additionally, similar material stiffness may reduce the mismatch in shrinkage rates during molding or post-processing, which mismatch may ultimately negatively impact lens optical resolution. In one embodiment, the haptic is stiffer than the optic. Moreover, the two materials have the same or similar refractive indices to reduce any unwanted glare or reflection from light passing across adjacent surfaces. A number of such embedded optics may be seen in U.S. Patent Publications 2008-0161913 and 2008-0161914.

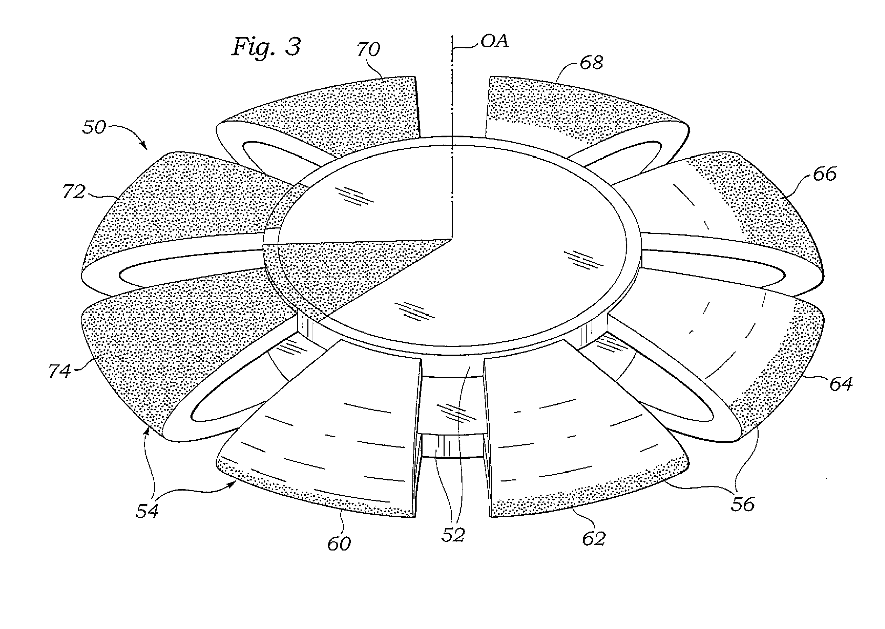

A number of intraocular lenses may be adapted to the concepts described herein to improve the accommodative performance of the haptic or IOL, such that compressive/tensile forces may be more efficiently transferred from the haptic to the optic. It will be understood that any combination of individual haptic or IOL features described herein, where appropriate, may be formed even if not explicitly described or shown. It is also noted that while described in relation to AIOLs, surface adherents or adhesives according to embodiments of the present invention may be used with a variety of types of IOLs or other ophthalmic lenses or devices (e.g., shunts). For instance, any IOL may benefit from a surface adherent or adhesive on its haptic and/or optic to fix the lens in position, enhance stability, and/or prevent PCO. This includes monofocal IOLs, multifocal IOLs, accommodation IOLs (AIOLs), phakic IOLs (PIOLs) and the like. For example, a thermo-reversible adhesive, which solidifies at body temperature, may be useful to initially attach an IOL and subsequently reverse the attachment temporarily to readjust the IOL position by flowing a cold BSS solution through the eye. Alternatively or additionally, an adherent comprising microfibers may be used. Figure 3 is a perspective view of an accommodative IOL 50 having a pair of axially spaced-apart optics 52 centered on an optical axis OA, and a plurality of convex haptic legs 54 connect the optics and radiating outward therefrom. The haptic legs 54 are configured to transmit forces from the surrounding capsular bag/zonules to alter the spacing between the optics 52.

In some embodiments, the AIOL 50 is symmetric across a midplane perpendicular to the optical axis OA such that there are matching legs 54 connected to each optic 52. Each pair of matching legs 54 joins may together at their outer ends in a convex outer curve 56 that may be configured to generally match the shape of a capsular bag of an eye into which the intraocular lens is inserted. As illustrated, there may be eight pairs of matching legs 54, though more and as few as three are contemplated. The convex outer ends of the haptic legs 54 provides a capsular bag-filling outer profile to the AIOL 50 that effectively couples the bag forces to the dual optics 52 to either axially expand or contract the spacing therebetween. That is, forces exerted on the outer ends of the haptic legs 54 are transmitted through the legs to cause the spaced optics 52 to move apart or toward each other, thus changing the dual lens focal length. Although movement between the two optics 52 may be configured to amplify a change in power (accommodative range), in some embodiments the AIOL 50 includes only one of the lenses 52, for example, to reduce criticality of alignment of the AIOL within the eye.

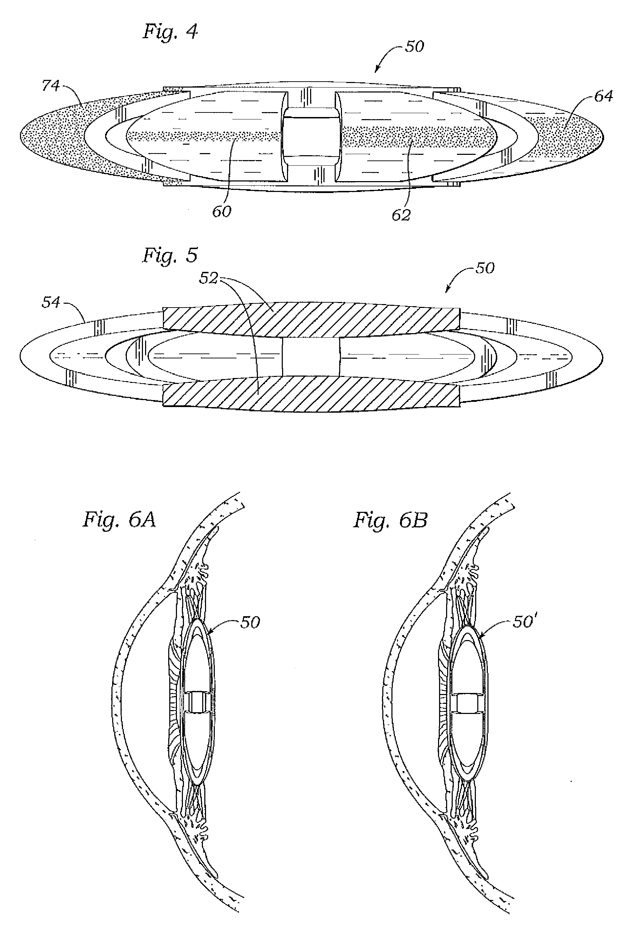

In accordance with the principles described herein, varying degrees of a surface adherent may be provided to the exterior of the AIOL 50. As seen in Figures 3 and 4, gradually larger regions of stippling are shown around the AIOL 50 and on succeeding haptic legs 54. A thin band of stippling 60 is shown on a leg 54 at the lower left in Figure 3, with gradually larger regions of stippling shown at 62-70 in a CCW direction around the AIOL 50. The largest region of stippling in this series at 70 covers the entire haptic leg 54. Continuing CCW, two other regions of stippling 72, 74 extend partway and all the way radially inward onto sectors on the optics 52 (the lower half shall be considered to be symmetric with the upper half, though such is not strictly necessary).

The regions of stippling 60-74 represent application locations for a number of different potential surface adherents or adhesives according to embodiments of the present invention. In general, surface adherents according to embodiments of the present invention are advantageously provide adhesion or adherence within a relatively short period of time (e.g., less than or equal to one second, less than 1 to 5 minutes, or less than 1 to 5 hours), help to prevent or control cell growth (e.g., PCO), are reversible, and/or otherwise provide mechanism for easily detaching a device after adhesion to a part of an eye. For instance, the regions of stippling 60-74 could be a thermo-reversible bioadhesive polymer such as polymerized N-isopropyl acrylamide (pNIPAM) (also known as NIPAAm (poly(N-isopropylacrylamide)). Alternatively, the regions of stippling 60-74 could comprise a plurality of microfibers, for example, having physical surface texturing designed to mimic the feet of certain lizards and insects. Each of these alternatives will be discussed in more detail below, including their favorable sites of application on the AIOL. Generally, the amount of surface adherent is sufficient to hold the AIOL in place under normal ocular forces after insertion into an eye. In some embodiments, reversible adhesion is provided by a substance that changes its adhesion characteristic with an intensity or wavelength of light, vibration of the adhesion interface, application or concentration of a chemical substance, exposure or intensity of an electric or magnetic field, or the like.

Polymeric systems that may modify adhesive properties in response to changes in the physical and chemical characteristics of the physiological medium are promising candidates to achieve reversible tissue adhesion. Several groups have explored the use of dynamic stimulus-responsive surface chemistries for cell patterning, thermo-active, electrical-active, and photoactive chemistries have been defined for cellular adhesion. In general, all of these chemistries operate under the same principle. These substances can be switched from a state that prevents cellular attachment to a state that promotes it. In the context of the present application, a reversible adhesive means one which can change state depending on certain stimulus, such as temperature for a thermo-reversible adhesive. Other possible stimuli include mechanical (e.g., vibration), light, radiation, chemical, or others.

A particularly useful composition for use in the present invention is a thermo-reversible bioadhesive polymer, such as a composition which is liquid at or below room temperature and forms a high viscosity layer or gel at body temperature. In certain embodiments, the thermo-reversible bioadhesive polymer has a first adherence value when at a temperature that is a predetermined amount below an average body temperature and has a second adherence value when at a temperature that is at or above the average body temperature, the second adherence value being greater than the first adherence value. The predetermined amount may be at least 2 degree Celsius, at least 3 degrees Celsius, at least 4 degrees Celsius, or at least 5 degrees Celsius, depending on factors such as the expected minimum temperature of a particular subject or a population of subjects (e.g., subjects having a certain age range or subjects likely to have a particular ophthalmic procedure or condition). The second adherence value will generally be at least twice that of the first adherence value, but may be at least 5 times, at least 10 times, or at least 100 times that of the first adherence value. The average body temperature may be 37 degrees Celsius.

Polymers having bioadhesive properties are for instance water-soluble cellulose derivatives, such as sodium carboxymethyl cellulose, and polyacrylic acids, which are used in many pharmaceutical preparations to improve the contact between drug and body. Improved uptake of ophthalmic drugs has been achieved by using vehicles containing viscosity-increasing polymers such as the cellulose derivatives, polyvinyl alcohol and polyvinylpyrrolidone. Thermogelling pharmaceutical preparations are described in U.S. Pat. Nos. 4,478,822, 4,474,751, 4,474,752 and 4,474,753, which refer to a drug delivery system which at room temperature has the properties of a liquid, but forms a semi-solid gel at human body temperatures. The compositions to be administered comprise 10 to 50% by weight of a polymer, which is a tetra-substituted derivative of certain diamines containing approximately 40 to 80% poly(oxyethylene) and approximately 20 to 60% poly(oxypropylene), as a drug delivery vehicle. In this system the gel transition temperature and/or the rigidity of the gel can be modified by adjustment of the pH. Other systems are known in which the gelling is induced by an increase in the amount of electrolytes or a change in pH. Further, certain water-soluble nonionic cellulose ethers in combination with a charged surfactant and optional additives in water have the property of being liquid at room temperature and forming a gel when warmed to body temperature, and the process is reversible.

A desirable thermo-reversible bioadhesive polymer for intraocular use is one that is nontoxic and biocompatible. Polymerized N-isopropyl acrylamide (pNIPAM) has been shown not to be toxic to neural tissue and is commonly used in cell and tissue cultures for its reversible cell adhesion properties. Previous reports showed that cells may be attached and detached from pNIPAM coated culture dishes without exhibiting any changes in morphology. Some studies show that pNIPAM has a lower critical solution temperature of 31°C in an aqueous environment. This may indicate that the reversible thermoresponsive adhesive or hydrogel (pNIPAM) exhibits decreased solubility or swelling in water as the temperature is increased, due to a phase transformation at the lower critical solution temperature. Thus, pNIPAM may be switched from a state that promotes cellular attachment to a state that prevents cellular attachment, as the temperature of the surface is decreased. A particular characteristic of this material is the ability to be adhesive at body temperature (37C) and not adhesive at room temperature. Various applications for such a bioadhesive are disclosed in US Patent Publication No. 2008-0140192, assigned to the University of Southern California.

The use of this type of thermo-reversible, or some other type of reversible, bioadhesive polymer with accommodating IOLs (AIOLs) may resolve two key issues currently challenging the use of AIOLs technologies (that is, prevention of LECs from proliferating ("PCO") and optimization of the coupling of the capsular bag to the AIOLs) by fully adhering the AIOL to the capsular bag once the AIOL is in place. Further, cold or room temperature saline could be injected at the device and/or into the capsular bag to release the adhesive to allow for re-position of the AIOL or its explantation.

If applied to a lens of an IOL or AIOL, the lens could be coated with the thermo-reversible bioadhesive polymer. In this case, the lens could be handled in a manner consistent with current standard cataract surgical procedures and inserted at operating room temperatures. Once the lens is implanted in the eye, the thermo-reversible polymer (such as pNIPAM) properties will allow the IOL to adhere to the capsular bag. The coating can be selective (specific areas of the AIOL) or on all surfaces of the AIOL as specified by the AIOL design to prevent LECs proliferation and to optimize capsular bag coupling. Also, as mentioned above, the adhesive may be reversible based on some other stimulus than a temperature change.

In one embodiment, a thermo-reversible bioadhesive polymer is coated on the exterior of the AIOL 50 prior to implant, and remains in a state that prevents cellular attachment (less adherent) while outside the body. After implant into the capsular bag, and a rise in temperature to match that of the body's, the thermo-reversible bioadhesive polymer undergoes a change of state to one that that promotes cellular attachment (more adherent). Post-surgically, if the AIOL 50 requires removal, replacement, or re-positioning, a cold saline or other such solution may be used to cause the thermo-reversible bioadhesive polymer to revert back to its less adherent state. Generally, the amount of thermo-reversible bioadhesive polymer is sufficient to hold the AIOL 50 in place under normal ocular forces after insertion into an eye.

With reference to Figures 3 and 4, one or more of the varying sizes shown of the stippled regions 60-74 may be reproduced on all haptic legs 54 of the AIOL 50. In one embodiment, the surface adherent is provided in thin bands, as in the small band 60, on the outer end of each haptic leg 54. One benefit from providing the thin surface adherent bands 60 is that the equatorial region of the haptic legs 54 adheres better within the area of the capsular bag where the zonular fibers attach to the bag. Also, providing adhesive between the haptic legs 54 and the capsular bag may prevent cell migration over these contact areas. Lens epithelial cell (LECs) often remain in the tight equatorial corner inside the capsular bag after attempts at removal. Adhering the haptic legs 54 to the capsular bag in these areas effectively eliminates any gap therebetween and thus inhibits further overgrowth. In some embodiments, a surface adherent is applied to selectively provide adhesion or adherence in a region where the zonules attach to the capsular bag, for example, to provide enhanced transfer of ocular forces to the capsular bag and AIOL. In such embodiments, other surface portions of the haptic and/or optic may be free of the bioadhesive polymer, for example, to allow relative motion between the capsular bag and the AIOL.

Alternatively, larger bands of a surface adherent as the band 62 may be used, or even larger bands as seen at 64-68, moving CCW around the AIOL 50. Ultimately, the entirety of each haptic leg 54 may be covered with the surface adherent, as seen at 70.

Depending on the effect on the optical performance, surface adherent may also cover a portion or the entire external surface of the optics 52 (or just one of the optics). For instance, region 72 shows the surface adherent extending inward beyond the corresponding haptic leg 54 and onto the outer rim of the optic 52. Likewise, region 74 shows the surface adherent extending inward beyond the corresponding haptic leg 54, over the outer rim of the optic 52, and onto the surface of the optic to its center. The stippling 74 has been drawn to indicate that if all of the sectors were so configured that the entire exterior surface of the AIOL 50 - that is, both the optics 52 and the haptic legs 54 - would be covered with a surface adherent. In some embodiments, a surface adherent is located on at least portions of one or both optics 52, but no, or little, surface adherent is located on the haptics legs 54, for example, to hold the AIOL in place and allow relative motion between the capsular bag and haptic legs 54.

As mentioned above, the regions of stippling 60-74 could be physical surface texturing designed to mimic the feet of certain lizards and insects. The ability of geckos, spiders and flies to adhere to seemingly shear surfaces has long fascinated researchers. For instance, geckos' exhibit a remarkable ability to stick to surfaces without the use of an adhesive substance (such as a polymer, etc.). Geckos foot surfaces are characterized by a plurality of microfibers that in some aspects are similar to synthetic microfibers. The adherent principle (i.e., adhesion through physical surface structure rather than exuded polymers, or other similar contact adhesives, etc.) is believed to be due to van der Waals forces.

A van der Waals force is the attractive or repulsive force between molecules (or between parts of the same molecule) other than those due to covalent bonds or to the electrostatic interaction of ions with one another or with neutral molecules. The term includes permanent dipole-permanent dipole forces, induced dipole-induced dipole forces, and instantaneous induced dipole-induced dipole (London dispersion forces). It is also sometimes used loosely as a synonym for the totality of intermolecular forces. Van der Waals forces are relatively weak compared to normal chemical bonds.

Through various molding processes and techniques, it is possible to mimic the microfiber structure found on gecko feet that provides such an adherent surface. Consequently, one "surface adherent" as defined herein is a surface having a plurality of microfibers thereon. Microfibers, in this context, may be defined as fibers having a diameter of between 3-5 microns (micrometers, µm). The microfibers may be provided in sufficient numbers/density over a particular area of the AIOL to provide adhesion between the AIOL and the surrounding capsular bag. This would provide immediate IOL-to-capsular bag fixation after implant as well as an easy detachment process through pealing. The microfibers may be provided in sufficient numbers/density over a sufficient area so as to hold the AIOL 50 in place under normal ocular forces after insertion into an eye.

For instance, microfibers may be molded in sufficient quantities along the perimeter of the haptic (such as in the thin bands 60, 62, or 64 in Figures 3 and 4) so that the existing capsular bag could adhere to them. Again, this adhesion will allow the haptic legs 54 to be more effectively pulled bringing the two optics closer (during dis-accommodation, reducing power) and pushed forcing the optics apart (during accommodation, increasing power). Locating these fibers primarily along the equator of the haptic legs 54 within the band where the zonular fibers attach to the bag provides excellent results in terms of improved force transfer during accommodation. Proper shape and sizing of the haptic structure would be necessary, as described below.

An exemplary discussion of a variety of microfiber configurations is given in U.S. Patent No. 7,344,617 to Dubrow.

Different embodiments of the invention comprise a range of densities (e.g., number of microfibers per unit area of a substrate to which microfibers are attached or associated) The number of microfibers per unit area can optionally range from about 1 microfiber per 10 micron² up to about 200 or more microfibers per micron²; from about 1 microfiber per micron² up to about 150 or more microfibers per micron²; from about 10 microfibers per micron² up to about 100 or more microfibers per micron²; or from about 25 microfibers per micron² up to about 75 or more microfibers per micron^{2.} In yet other embodiments, the density can optionally range from about 1 to 3 microfibers per square micron to up to approximately 2,500 or more microfibers per square micron

In terms of individual fiber dimensions, it will be appreciated that by increasing the thickness or diameter of each individual fiber, one will again, automatically increase the area of the fiber that is able to make intimate contact with another surface, whether such contact is with a fiber that is directly orthogonal to the second surface or is parallel or tangential with that other surface The fiber thicknesses are optionally between from about 3-5 microns. Choice of microfiber thickness can also be influenced by compliance of such microfibers (e.g., taking into account that microfiber's composition, etc.) Thus, since some compositions can produce a less compliant microfiber at greater diameter such changes can optionally influence the choice of microfiber diameter.

In the case of parallel or tangential contact between fibers from one surface and a second surface, it will be appreciated that by providing fibers of varying lengths, one can enhance the amount of contact between a fiber, e.g., on an edge, and the second surface, thereby increasing adhesion. Of course, it will also be understood that for some fiber materials, increasing length may yield increasing fragility Accordingly, fiber lengths will typically be between about 30 microns or less up to about 130 microns.

In terms of the AIOL 50 illustrated in Figures 3-5, the microfibers mimicking gecko feet are desirably provided only on the haptic legs 54, and not on the optics 52, as the physical surface irregularities thus presented may interfere with the optical transmission quality. However, as with other surface roughening treatments, microfibers may be provided on an outer portion of the optics 52 without deterioration of vision, such as in regions like 72 around the AIOL 50.

It is also possible to combine different surface adherents on a single lens, such as a bioadhesive (e.g., pNIPAM) and microfibers (e.g., gecko feet). For example, microfibers may be provided on the IOL haptics, while a bioadhesive is coated on at least a portion of the optic for lower interference with the optical transmission through the lens. One contemplated embodiment is for microfibers on the IOL haptics to be coated with a bioadhesive which is reversible so as to be relatively thick at room temperature and liquid at body temperature. This configuration prevents the microfibers from sticking to surrounding structures and instruments prior to implant, but exposes the microfibers after implant for good adhesion or adherence to the capsular bag.

Figure 6A and 6B are vertical sectional views through an eye showing the implanted exemplary AIOL of Figures 3-5 in two states of accommodation. In Figure 6A the zonules pull on the equatorial region of the capsular bag and cause elongation of the AIOL 50, such that the two optics 52 are brought closer together, thus decreasing the optic power. In Figure 6B the zonules push radially inward on the equatorial region of the capsular bag and cause a squeezing of the AIOL 50', such that the two optics 52 are separated in the axial direction, producing an increase in the power of the optic. Again, these reactions to the muscle movement of the zonules are accentuated by the intimate and adherent contact between at least the equatorial region of the exemplary AIOL haptics with the capsular bag.

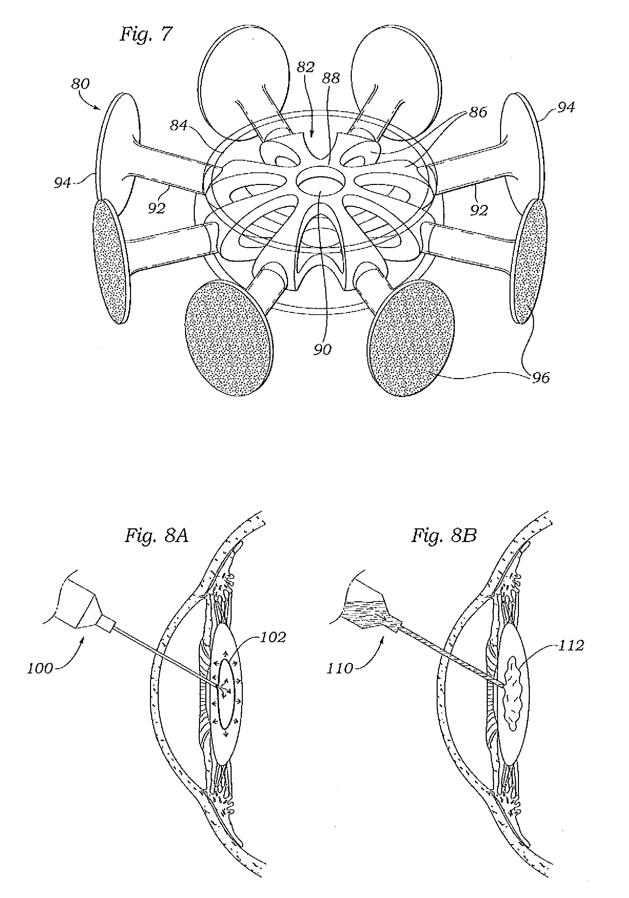

Another embodiment of AIOL 80 into which the benefits of the present application may be incorporated is shown in Figure 7. The AIOL 80 includes a haptic 82 embedded within a relatively softer optic 84. As was described in U.S. Patent Publications 2008-0161913 and 2008-0161914, mentioned above, various AIOL embodiments provide a haptic partly embedded within an adjustable or accommodative central optic that are also within the scope of embodiments of the present invention. The haptic transmits forces to alter at least one of the shape and the thickness of the adjustable optic. The materials of the haptic 82 and optic 84 may have similar or equivalent refractive indices at one or more wavelength so as to reduce glare or reflection from light passing across haptic/optic interfaces.

The haptic 82 includes a plurality of spoke-like legs 86 that each terminate at an outer end in a convex surface and include bifurcated segments that converge in two axially-spaced inner rings 88 surrounding central apertures 90. The resulting structure is a series of vaulted legs 86 joined in the middle. Each leg 86 further includes a cylindrical strut 92 extending outward from its outer end that ends in an enlarged disk-shaped head 94. Each strut 92 and head 94 combination resembles a combustion engine cylinder valve.

The outermost face of each head 94 has a surface adherent 96 thereon, indicated by stippling. Although the entire outer face of each head 94 is shown covered with the surface adherent 96, only portions thereof may be covered, such as, for instance, the peripheral edge. The AIOL 80 of Figure 7 may rely on the same capsular bag fixation technique as described above, with adhesion along the capsular bag equator to push and/or pull on the single optic 84. In this case, instead of relying on power change from dual optic movement, the forces are transferred via the haptic 82 towards the center of the soft optic body 84, thus inducing a change in power by changing the shape or curvature of the optic surface. In the illustrated embodiment, each head 94 has an oval shape; however other shapes may be used, such as circular, rectangular, triangular, or the like. The shape and/or orientation of each outer face may be the same or different from that of the remaining outer faces. In some embodiments, adjacent faces may be configured to form interlocking or complementary shapes. For example, one outer face may be a triangular or arrowhead shape pointing in the anterior direction, while adjacent outer faces are a triangular or arrowhead shape pointing in the posterior direction. In general, the outer faces may be configured or sized to increase or provide at least a minimum amount of adhesion to the capsular bag or eye. The outer faces may also be configured to affect how force from the capsular bag wall is transmitted to the optic 84. For example, a larger area and/or more adhesive may be provided on the anterior side than the posterior side of one or more outer faces (or visa versa), so as to provide a torque on, or vaulting of, the optic 84.

The faces of each head 94 in the illustrated embodiment are flat. Such flat faces may be beneficial during application of an adhesive material to the faces. For example, microfibers generally provide a more favorable adherent when applied normal to the surface to which they are applied. In such embodiments, the material and/or thickness of may be selected to allow the heads 94 to easily conform to the shape of the capsular bag into which the AIOL is placed. Alternatively, the faces may be curved or configured before insertion to fit the capsular bag shape (either before or after application of an adherent to the faces).

Various configurations of surface adherent 96 are contemplated for the AIOL 80, including an adhesive such as the thermo-reversible bioadhesive polymer described above, or microfibers. In the case of microfibers, the fibers would desirably be formed normal to the oval-shaped haptic heads 94.

It will be understood that the AIOL embodiments of Figures 3 and 7 are only two of a myriad of lens designs that could benefit from direct attachment to the capsular bag using the surface adherents described herein. Again, the principle attachment area would at least be along the equator of the capsular bag, though other designs may benefit from anterior or posterior capsular bag attachments as well.

Figures 8A and 8B show a modified technique for implanting an injectable polymer AIOL in accordance with the principles described herein. Injectable AIOLs are known in the art, such as in U.S. Patent Nos. 4542542, 4608050, 6589550, 6598606, and 7182780.

In general, these patents describe techniques for removing a cataracteous and/or presbyopic natural lens from the capsular bag of the eye and replacing it by a lens-forming liquid material injected directly into the capsular bag. The liquid material is a partially polymerized material, which can undergo a curing process in the eye and thereby form a solid lens implant. The lens implant acts as a substitute for the natural lens and aims to substantially restore the features of the natural lens of the young eye. The defective natural lens matrix can be removed by a conventional surgical method involving an ultrasound probe, such as a phacoemulsification method involving aspiration. In order to facilitate the removal of the lens matrix and refilling with lens forming liquid material, a capsulotomy, i.e. a capsulorhexis, is prepared from a circular or essentially circular capsulotomy in the capsular bag wall, typically with a diameter of from about 0.5 to about 2.5 mm. An injection syringe needle is inserted through an incision in the eye and through the capsulorhexis into the capsular bag so the lens-forming liquid material can be injected into the capsular bag.

One technique is to "coat" the capsular bag with a layer of the thermo-reversible polymer just prior to the AIOL implantation/capsular bag filling with polymer material injected into it (for Injectable IOLs) during the cataract surgery procedure. This can be achieved for example by manually applying the thermo-reversible polymer by the surgeon using adjunct instrumentation, by implanting a temporary IOL, device or "bag-filling balloon" that will transfer the layer to the capsular bag and then be removed. Once again, a reversible adhesive in general may be used, the thermo-reversible polymer being particularly useful.

For instance, Figure 8A illustrates a cannula 100 inserted into the previously evacuated capsular bag space and inflating a balloon 102. The balloon 102 has been coated with a favorable bioadhesive, such as pNIPAM as described above. Eventually, the balloon 102 fills the space within the capsular bag and the adhesive transfers to the bag. The balloon 102 is then deflated and the cannula 100 removed.

Subsequently, the surgeon advances the needle of a syringe 110 into the capsular bag and injects a polymer material 112 that will form the AIOL. The material 112 fills the space within the capsular bag and comes into intimate contact with the adhesive previously applied. This arrangement fully adheres the AIOL to the capsular bag and effectively couples the forces of the natural accommodative mechanism of the eye to the AIOL to maximize accommodation amplitude for years with no expected degradation over time. Full adhesion of the AIOL/Injectable Polymer to the capsular bag also prevents or reduces lens epithelial cell (LECs) migration over those areas.

Rather than injecting an amorphous mass into the capsular bag, an injectable IOL could be encapsulated within a flexible structure like a balloon which is then inflated to fill the capsular bag. Such a configuration may be better received by the immune system of the eye. In such a case, an adhesive layer may be provided on the outside of balloon rather than on the inside of the capsular bag. The balloon could be partly inflated prior to implant or fully inflated after implant, though obviously the latter reduces the size of the capsulotomy necessary.

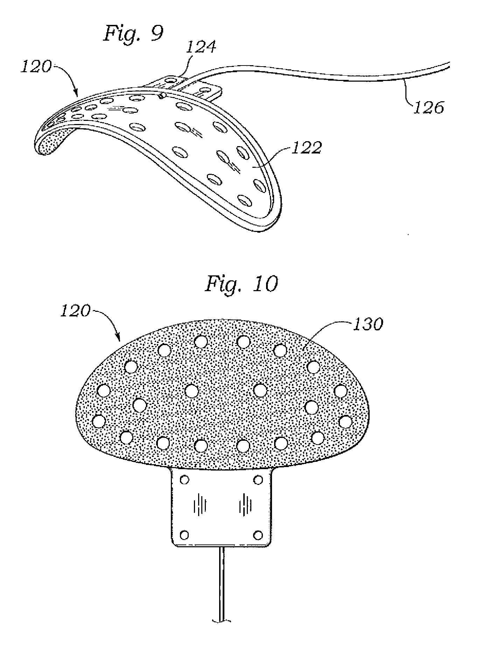

Another use for the surface adherents described herein is with glaucoma shunts, such as shown at 120 in Figures 9 and 10. The shunt 120 includes a large plate 122, which may be curve to conform around the sclera and/or may include a small tab 124 extending from one side. An elongated flexible drainage tube 126 opens at one end over the plate 122, and another end is free. The free end may be inserted into the inner fluid chamber of the eye to initiate fluid drainage therefrom.

The underside of the plate 122 is covered with a surface adherent, shown as stippling in Figure 10. Again, the entire surface may be covered with adhesive material, or at least those portions in between fenestration holes. Alternatively, only a peripheral edge or some other portion of the plate underside may be covered. Additionally or alternatively, the tab 124 and/or the flexible drainage tube 126 may be partially or completely covered with adhesive material. When adhesive material covers all or portions of the plate 122, the surface adherent will bond to the sclera. In any event, the use of an adhesive material according to embodiments of the present invention may eliminate or reduce the need for temporary sutures. In some embodiments, the use of an adhesive material may also eliminate or reduce the need for the tab 124 that typically was used for a suture anchor. The surface adherent for the glaucoma shunt 120 may be a microfibers material and/or thermo-reversible polymer as described above.

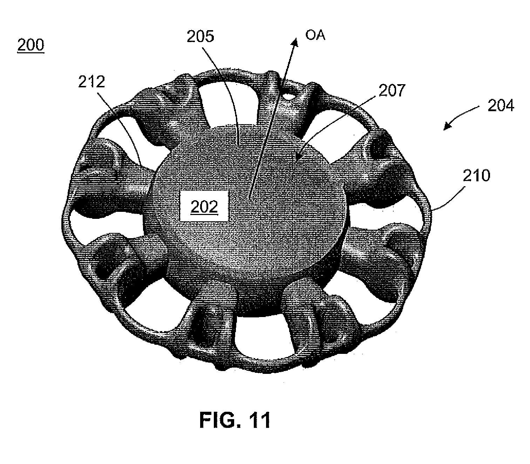

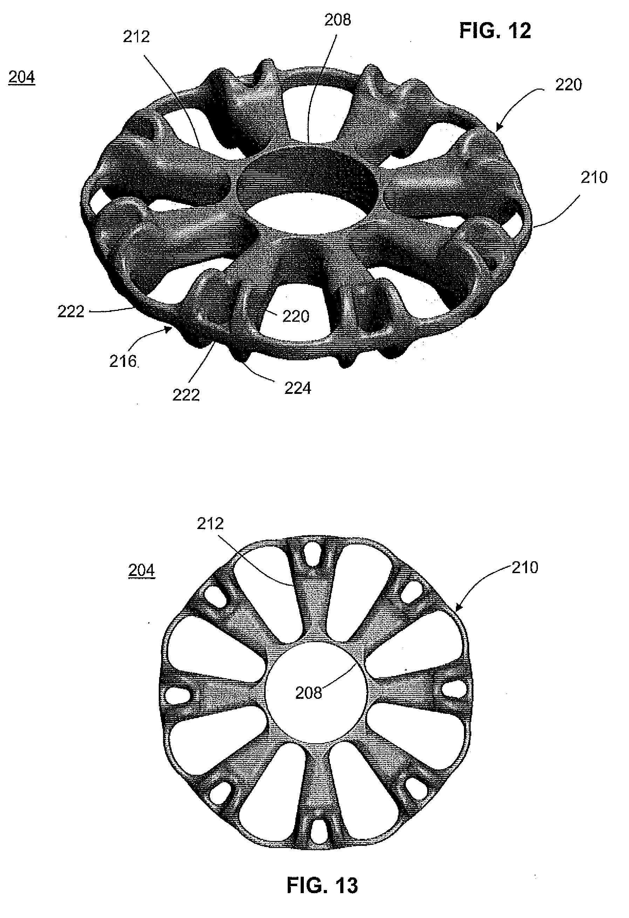

Referring to Figures 11-12, an AIOL 200 according to an embodiment of the present invention is shown that comprises an adjustable optic or optic body 202 disposed about an optical axis OA and a haptic or support structure 204 configured to transfer an ocular force from a human or animal eye to optic 202 so as to produce a range of powers in response to an ocular force. Optic 202 includes an anterior face 205 and a posterior face 206 that together define a clear aperture 207. Haptic 204 includes an inner structure 208 and an outer structure 210 and an intermediate structure 212 that may be in the form of a plurality of arms. Arms 212 connect or couple structures 208, 210 to one another so as to transfer the ocular force to changing the shape and/or axial location of optic 202, thereby providing a change in optic power and/or focal plane location of optic 202. Outer structure 210 comprises an outer face 216 configured for engaging the interior face of a capsular bag of an eye, the outer face 216 includes an equatorial region 222, as well as an anterior region 220 and a posterior region 224 disposed on opposite sides of equatorial region 222 in a direction along optical axis OA. At least portions of one or more of regions 220, 222, 224 may include one or more of an adherent or adhesive discussed above herein for attachment to the capsular bag.

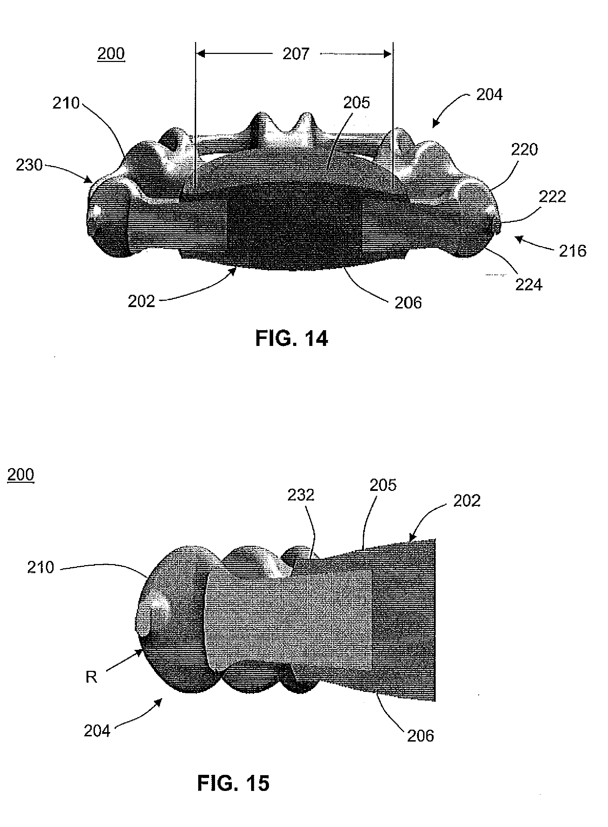

Referring to Figures 14 and 15, outer structure 210 notably has a peripheral region 230 that is generally arcuate in cross-section, for example, to engage a relatively large portion of the capsular bag. In some embodiments, outer structure 210 has an axial thickness in the vicinity of peripheral region 230 that is from 1.8 millimeters to 2.2 millimeters or about 2.0 millimeters (e.g., 2 millimeters plus or minus 0.1 millimeters). It has been discovered that the relatively large axial thickness of peripheral region 230 is effective in transferring much of the forces produced by the capsular bag and/or zonules of an eye, since capsular bag is engaged over a large axial extent. Thus, outer structure 210 engages a large extent or area of capsular bag, while also providing skeletal structure with a relatively low mass. The low mass of outer structure 210 results in a relatively low stiffness, thus allowing it to conform to changes in the shape of capsular bag during accommodation. This, in turn, allows more of the forces produced by the changing shape of capsular bag to be coupled into haptic 204 and transferred into changing the shape and optical power of optic 202.

Peripheral region 230 has an arcuate shape in a plane parallel to, and passing through, the optical axis OA that is convex. The arcuate shape is characterized by a radius of curvature R. In certain embodiments, radius of curvature R is equal to a radius of curvature of an average capsular bag of a population. For example, radius of curvature R may be 1.13 millimeters plus or minus 0.02 millimeters. In certain embodiments, radius of curvature R is greater than a radius of curvature of an average capsular bag of a population. For example, radius of curvature R may be 1.16 millimeters plus or minus 0.02 millimeters or greater than 1.16 millimeters.

Arms 212 protrude or extend into, or inside of, the clear aperture 207 of optic 202. As used herein, the term "clear aperture" means the area of a lens or optic that restricts the extent of a bundle of rays from a collimated source or a distant light source that can imaged or focused by the lens or optic. The clear aperture is usually circular and is specified by its diameter. In some embodiments, the clear aperture has the same or substantially the same diameter as the optic. Alternatively, the diameter of the clear aperture may be smaller than the diameter of the optic, for example, due to the presence of a glare or PCO reducing structure disposed about a peripheral region of the optic.

Since inner structure 208 and the proximal ends of arms 212 are located inside optic 202 and within the clear aperture thereof, at least these portions of haptic 204 are beneficially transparent or nearly transparent, so that it does not substantially block or scatter any light transmitted through optic 202. In addition, these portions of haptic 204 may have a refractive index that matches the refractive of optic 202 material so that interfaces between optic 202 and haptic 204 do not produce significant reflections or refractions that might produce scattered light within the eye, which might appear as a glare or haze to the patient.

A numerical example may be used to illustrate the effect of mismatch of refractive indices on reflected power. For a planar interface at normal incidence between air (refractive index of 1) and glass (refractive index of 1.5), 4% of the incident power is reflected at the interface. For such an interface between air and glass, there is no attempt to match refractive indices, and this 4% reflection will merely provide a baseline for comparison. If, instead of 1 and 1.5, the refractive indices differ by 4%, such as 1.5 and 1.56, or 1.5 and 1.44, there is a 0.04% reflection, or a factor of 100 improvement over air/glass. Finally, if the refractive indices differ by only 0.3%, such as 1.5 and 1.302 or 1.5 and 1.495, there is a 0.00028% reflection, or a factor of over 14000 improvement over air/glass. In practice, tolerances such as the 0.3% case may be achievable, and it is seen that a negligible fraction of power may be reflected at the interface between a haptic and an optic whose refractive indices differ by 0.3%. Note that the above base value of 1.5 was chosen for simplicity, and that haptic 204 and optic 202 may have any suitable refractive index.

Thus, the refractive indices of optic 202 and at least portions of haptic 204 inside optic 202 are equal or essentially the same. For the purposes of this document, "essentially the same" means that their refractive indices are equal to each other at a wavelength within the visible spectrum (i.e., between 400 nm and 700 nm). Note that haptic 204 and optic 202 may optionally have different dispersions, where the refractive index variation, as a function of wavelength, may be different for the haptic and the optic. In other words, if the refractive indices of haptic 204 and optic 202 are plotted as a function of wavelength, they may or may not have different slopes, and if the two curves cross at one or more wavelengths between 400 nm and 700 nm, then the refractive indices may be considered to be essentially the same or essentially equal.

Referring to Figure 15, in some embodiments, the relatively thick optic 202 comprises a peripheral region 232 that includes, in cross section, a counter taper that is configured to reduce glare from light incident on optic 202. The counter taper may have an angle from the horizontal plane that is from -3 degrees to -7 degrees. Thus, the angle formed in cross section at the juncture of peripheral region 232 and other portions of the adjacent optic 202 surface is less than 180 degrees.

In order to provide adhesion or adherence of outer face 216 to the capsular bag, an adhesive, such as a thermo-reversible bioadhesive polymer, may be applied to any or all regions 220, 222, 224 of outer face 216 of support structure 204, for example, in a manner similar to that shown on one or more of legs 54 illustrated in Figure 3. In some embodiments, adhesive is applied over all, or is applied over at least 80 percent or at least 90 percent of, outer face 216 in order to transfer force form a larger area of the capsular bag and/or zonules. In other embodiments, adhesive is applied only to only one of regions 220, 222, 224, for example, to allow greater flexibility of the capsular bag between accommodative and disaccommodative configurations. Depending of the stiffness of optic 202, the geometry of support structure 204, and how adhesive is applied to outer face 216, support structure 204 may change the shape of optic 202 and/or translate optic 202 along optical axis.

In certain embodiments, AIOL 200 is configured or selected to have an accommodative bias when placed inside the capsular bag of a subject eye. An accommodatively biased AIOL can advantageously increase the accommodative range over that of an AIOL that is disaccommodatively biased, since the latter relies on forces produced by the resiliency of the capsular bag to push arms 212 radially inward and thereby decreased the radius of curvature of one or both optic faces 205, 206. The forces produced by capsular bag resiliency may be relatively weak compared to the ocular forces produced by ciliary muscle and connective zonules when the ciliary muscle relaxed to provide distant vision. In addition, an accommodatively biased AIOL may advantageously less sensitive to the fit between support structure 204 and the inner wall of the capsular bag than is a disaccommodatively biased AIOL. For example, if a disaccommodatively biased AIOL is too small, the capsular bag may apply little or no force to the IOL. Thus, the IOL may provide little or no accommodative power change. By contrasts, if the AIOL is accommodatively biased, at least portions of support structure 204 may adhere to the capsular bag so that disaccommodation or negative accommodation is produced as the zonules stretch arms 212 radially outward to produce a tensile force. When using an accommodatively biased AIOL 200, an adhesive according to an embodiment of the present invention may be advantageously applied to all or portions of support structure 204. Such an adhesive can help maintain contact between the inner wall of the capsular bag and the support structure 204 as arms 212 of support structure 204 are stretched and put in tension as arms 212 are pulled radially outward by the capsular bag, zonules, or ciliary muscle of the eye.

In other embodiments, AIOL 200 is configured or selected to have an intermediate bias when placed inside the capsular bag of a subject eye, wherein AIOL 200 provides intermediate vision when in a natural or unstressed state. As with the accommodatively biased version of AIOL 200, an adhesive may be advantageously applied to all or portions of support structure 204 to help maintain contact between the inner wall of the capsular bag and the support structure 204 when the arms 212 are stretched to produce a tensile force as the capsular bag, zonules, or ciliary muscle pull arms 212 radially outward. In addition, an adhesive may help to maintain stability of AIOL 200 as the resiliency of the bag produces an ocular force that compresses arms 212 that increases the curvature and optical power of optic 202 to provide near vision.

In certain embodiments, performance of an accommodatively biased or intermediately biased AIOL 200 may be enhanced by selectively applying an adhesive over at least portions of one or two of regions 220, 222, 224 of outer face 216. For example, adhesive may be selectively applied to all or portions of posterior region 224, while no adhesive is applied to anterior and equatorial regions 220, 222. Alternatively, anterior and/or equatorial regions 220, 222 may use a different adhesive or some of the same adhesive, but applied to smaller surface areas and/or in smaller concentrations per unit surface area. Thus, posterior region 224 will have an adhesion that is greater than an adhesion of anterior and equatorial regions 220, 222. In such embodiments, when the capsular bag, zonules, and/or ciliary muscle pulls on support structure 204, more radially outward force is applied to the posterior side of AIOL 200 than to the anterior side, thereby providing a torque that causes optic 202 axially move in a posterior direction to decrease the distance between the retina and optic 202. Thus, as outer structure 210 is pulled radially outward, two action may occur: (1) the optic 202 distance form the retina decreases and (2) the power of optic 202 decrease as one or both faces 205, 206 becomes flatter. Both these actions favorably work together to provide distant vision.

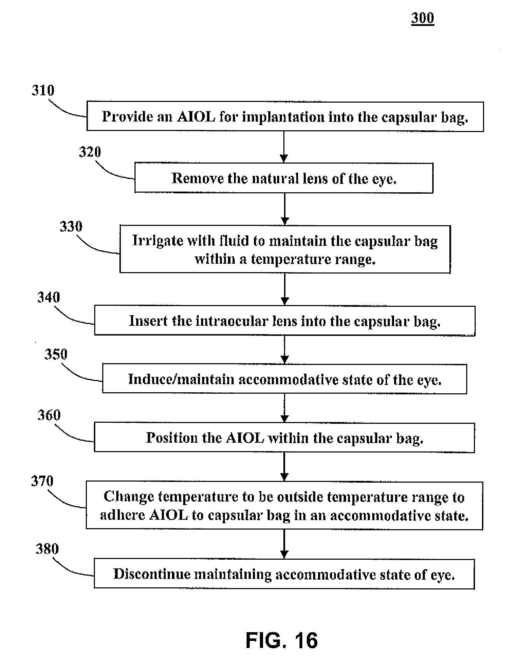

Referring to Figure 16, in certain embodiments, a method 300 of implanting an accommodative IOL is used to provide accommodative vision to an eye of a human or animal subject. Method 300 comprises an element 310 of providing AIOL 200 for implantation into the capsular bag of the subject. Method 300 also comprises an element 320 of removing the natural lens of the eye and an element 330 of providing and maintaining a temperature in the capsular bag and/or of AIOL 200 that is within a first temperature range. Method 300 further comprises an element 340 of placing or inserting the intraocular lens into the capsular bag. Method 300 additionally comprises an element 350 of inducing and maintaining an accommodative state of the eye by contracting the ciliary muscle and/or zonules of the eye. Method 300 also comprises an element 360 of positioning AIOL 200 within the capsular bag and/or of AIOL 200 while the temperature in the capsular bag is within the first temperature range. Method 300 further comprises an element 370 of adhering at least a portion of outer face 216 of support structure 204 to the inner wall of the capsular bag by changing the temperature inside the capsular bag and/or AIOL 200 to a temperature that is outside the first temperature range. Method 300 also comprises an element 380 of discontinuing the inducing of the accommodative state.

Element 310 includes providing AIOL 200; however, method 300 may be utilized with other IOLs or ophthalmic devices, especially other AIOLs (e.g., such as those illustrated in Figures 2, 3, 7, and 8). For simplicity, method 300 will be discussed in the context of AIOL 200. AIOL 200 includes an adhesive, for example, a thermo-reversible bioadhesive polymer or some other type of adhesive for which the degree of adhesion may be varied after implantation within the eye (e.g., a polymer material whose adhesion changes when exposed to ultraviolet or blue light radiation or some other initiator). The adhesive material is generally disposed over portions of an IOL that are in contact with the equatorial regions of the capsular bag and/or regions adjacent to or near the equatorial regions of the capsular bag. Additionally or alternatively, adhesive material is disposed over at least portion of one or both faces 205, 206 of optic 202.

In element 320, the natural lens of the eye is removed from the capsular bag, for example, using phacoemulsification and/or some other technique for cutting, emulsify, or otherwise modifying the natural lens to a state to facilitate removal from the eye (e.g., using a laser). In some embodiments, the natural lens has already been replaced by an IOL or AIOL, wherein element 320 alternatively includes removal of the old IOL in preparation for replacement by AIOL 200.

Element 330 includes providing and maintaining the evacuated capsular bag cavity and/or AIOL 200 within a predetermined or desirable first temperature range in which the adhesive provides a relatively low adhesion, or no or essentially no adhesion, between outer face 216 of AIOL 200 and the inner wall of the capsular bag. For example, the capsular bag may be irrigated with an irrigation fluid, such as a saline solution, having a temperature that is within or near the first temperature range. In some embodiments, range includes temperatures that are below a transition temperature of an adhesive. As used herein, the term "transition temperature" is a temperature above which an adhesive or adherent adheres to tissue of the eye and below which it does not adhere to tissue of the eye. In some embodiments, range includes temperatures that are lower than that of an average body temperature of the subject or a given population of human or animal subjects. For example, the temperature within the capsular bag may be maintained at temperatures that that are at least 1 degree Celsius, 1.5 degrees Celsius, 2 degrees Celsius, 3 degrees Celsius, or 4 degrees Celsius below an average body temperature, depending on the adhesive properties and physiological factor of the subject. In the case of a human subject, the average body temperature may be considered to be 37 degrees Celsius and the temperature range may include temperatures that are less than or equal to 36 degreed Celsius, 35.5 degrees Celsius, 35 degrees Celsius, 34 degrees Celsius, or 33 degrees Celsius.

In such embodiments, the adhesive may be a thermo-reversible bioadhesive polymer that has a low adhesion, essentially no adhesion, or no adhesion to the inner wall of the capsular bag when maintained at a temperature that is within the first temperature range. However, when the thermo-reversible bioadhesive is outside the first temperature range, the adhesion of the thermo-reversible bioadhesive increases so as to secure the AIOL 200 to the capsular bag. For example, the thermo-reversible bioadhesive may have a relatively high adhesion when at the average body temperature or above, and may maintain a relatively high adhesion at temperatures that are slightly below the average body temperature, for example, at temperatures that are 1 degree, 2 degrees, or 3 degrees below the average body temperature.

In other embodiments, the temperature range may be above the average body temperature, wherein a thermal adhesive may be used on AIOL 200 that has a relatively low adhesion at temperatures above the average body temperature and a relatively high adhesion at or near the average body temperature. In some embodiments, an adhesive is used whose adhesion depends on some parameter other than temperature, for example, a photopolymer whose adhesion increases as exposure time to radiation is increased (e.g., radiation within the ultraviolet band of the electromagnetic spectrum and/or within the blue band of visible light). In such embodiments, element 330 may be eliminated or replaced by controlling some other parameter besides temperature.

Element 340 includes placing or inserting AIOL 200 inside the capsular bag, for example, using forceps or a lens inserter. AIOL 200 may be inserted with the adhesive material applied to AIOL 200 prior to implantation within the eye, for example, at a manufacturing facility, at a distributor facility, or at a hospital or clinic. Additionally or alternatively, some or all of the adhesive material may be applied after AIOL 200 has been implanted within the eye or capsular bag.

An accommodative state of the eye is induced or maintained in element 350 once AIOL 200 has been located inside the capsular bag. Alternatively, the accommodative state is induced prior to insertion of AIOL 200. In such embodiments, an accommodative state may be induced before or after making an incision in the eye, or before or after removing the natural lens. The induced state of accommodation may be produced by the introduction of a chemical compound either topically or inside the eye. In some embodiments, the chemical compound has muscarinic components, such as muscarinic agonists and muscarinic antagonists, to assist or facilitate the action of the ciliary muscle and/or associated zonules so that the AIOL in the eye is moved to provide either positive accommodation (near or intermediate vision) or negative accommodation (distant vision).

In some embodiments, the accommodative state of the eye is suitable for providing near or intermediate vision and AIOL 200 is an accommodatively biased IOL that is configured to provide either near or intermediate vision after a surgical procedure when AIOL 200 is in a natural or unstressed state or condition. In such embodiments, arms 212 are stretched radially outward to produce a tensile force that decreases the optical power of optic 202 to provide distant vision. Alternatively, a near or intermediate vision state of the eye is induced or maintained when the AIOL 200 has a disaccommodatively bias. In such embodiments, the induced state of the capsular bag may be used to tighten the capsular bag around outer face 216 of support structure 204, which in turn may help to increase the adhesion between outer face 216 and the inner wall of the capsular bag.

In other embodiments, the accommodative state of the eye is negative accommodation to insure that capsular bag has a shape suitable for providing vision after a surgical procedure. In such embodiments, AIOL 200 will generally have a disaccommodative bias, since the bag is configured to provide distant vision when adhesion is produced between the capsular bag and AIOL 200.

In element 360 of method 300, AIOL 200 is positioned within the capsular bag, for example, to provide alignment between optic 202 of AIOL 200 and the optical axis of the eye. During this part of the procedure, the relatively low adhesion between the capsular bag and support structure 204 allows AIOL 200 to be manipulated with relative ease. In some embodiments, the eye may be in a relatively disaccommodated state relative to the accommodative bias of AIOL 200, which may permit AIOL 200 to be more easily moved or manipulated within the capsular bag. For example, if AIOL 200 has an accommodative bias or an intermediate bias, the capsular bag may be maintained in a state of disaccommodation or negative accommodation, wherein the capsular bag is stretched by the ciliary muscle. This arrangement may facilitate manipulation of AIOL 200 within the capsular bag. In such embodiments, element 350 may be delayed or only partial implemented until AIOL 200 is at or near a desired position or orientation, at which time the eye is more fully accommodated so that the capsular bag conforms to and/or tighten around AIOL 200.

In element 370 of method 300, the temperature inside the capsular bag and/or of AIOL 200 is changed to a temperature that is outside the first temperature range, for example, by changing the temperature of an irrigation fluid flowing through the eye. For example, the temperature may be change to a temperature that is equal to or near an average temperature or a temperature of the subject (e.g., by discontinuing the flow of irrigation fluid through the eye). When a thermal adhesive is used, the change in temperature causes the adhesion between the capsular bag and at least portions of the support structure 204 to increase so that AIOL 200 is securely attached to the capsular bag. In order to increase pressure of the capsular bag inner wall against AIOL 200, the accommodative state of the eye may also be changed from the state maintained in element 350. This change in accommodative state of the eye may occurs prior to, during, or after a temperature change is induced. In embodiments where element 330 includes one or more other control parameters in addition to or besides temperature, element 370 may include controlling such parameters to provide a change in adhesion between AIOL 200 and the capsular bag. In embodiments where element 330 is eliminated from method 300, some other parameter my be altered to change adhesion between AIOL 200 and the capsular bag, for example, a radiation within an appropriate wavelength band may be applied to a photopolymer adhesive to increase adhesion.

Adhesion of AIOL 200 to the capsular bag may be maintained at a level to maintain a high degree of contact area when AIOL 200 is pulled radially by the ciliary muscle or zonules during disaccommodation or negative accommodation of the eye. In some embodiments, the adhesion of one or more of anterior region 220, equatorial region 222, or posterior region 224 of the outer face 216 is an amount sufficient to maintain a constant contact area between the one or more regions 220, 222, 224 and the inner wall of the capsular bag when an ocular force radially stretches an outer diameter of support structure 204 by a specified or predetermined amount For example, the adhesive and/or outer face 216 surface may be configured such that constant contact is maintained when the support structure 204 outer diameter is stretched or expanded by 5 percent, 10 percent, or 20 percent from a natural or unstressed state of AIOL 200. Additionally or alternatively, the adhesive and/or outer face 216 surface may be configured such that constant contact is maintained when the support structure 204 outer diameter is stretched or expanded by a force sufficient to change the power of AIOL 200 from a power that provides the eye with intermediate or near vision to a power that provides the eye with near vision. Additionally or alternatively, the adhesive and/or outer face 216 surface may be configured such that constant contact is maintained when the support structure 204 outer diameter is stretched or expanded by a force sufficient to reduce the power of AIOL 200 by at least 2 Diopter, by at least 3 Diopters, or by at least 4 Diopter. In other embodiments, the contact area between the one or more regions 220, 222, 224 and the inner wall of the capsular bag is somewhat reduced by some amount when an ocular force radially stretches an outer diameter of support structure 204 by one of the specified amounts. For example, the contact area between the one or more regions 220, 222, 224 and the inner wall of the capsular bag may be reduced by less than 10 percent, less than 20 percent, or less than 30 percent when an ocular force radially stretches an outer diameter of support structure 204 by one of the specified amounts.

In addition to securing IOLs in the eye, such as in the capsular bag, certain of the adhesives described herein are suitable for other ophthalmic uses. For instance, as described previously the procedure for injecting polymer type of IOL involves formation of an essentially circular capsulotomy in the capsular bag wall, typically with a diameter of from about 0.5 to about 2.5 mm. One application of the reversible adhesives described herein is in plugging this capsulorhexis. A small amount of pNIPAM, for example, deposited into the capsulorhexis may be sufficient to close it. The instrument that deposits the adhesive may include some form of shaper that spreads the adhesive in a thin layer across the capsulorhexis, and may linger for a sufficient time for a thermo-responsive adhesive to set up. Alternatively, a light-sensitive adhesive may be used which sets up on absorbing light from an LED or other such source.

Another potential application for the adhesives described herein is in fixing capsular bag ruptures after implant of an IOL, PIOL or AIOL. Again, an adhesive responsive to an external stimulus such as a temperature change may be deposited at a tear in the capsular bag and held in place long enough to gel or otherwise harden.

Still another application is in repair of at least small tears between the zonules and the capsular bag.

Finally, the adhesives may be used to seal a surgical incision through the cornea/sclera after cataract surgery.

While the invention has been described in various embodiments, it is to be understood that the words which have been used are words of description and not of limitation. Therefore, changes may be made within the appended claims without departing from the true scope of the invention.

## Claims

1. An accommodating intraocular lens (50, 200) for implantation into a capsular bag of an eye, comprising:
an adjustable optic body (21, 52, 202) disposed about an optical axis (OA) and including an anterior face (205) and a posterior face (206), the faces (205, 206) defining a clear aperture (207); a support structure (23, 204) adapted to transfer an ocular force from a capsular bag to the optic body (21, 52, 202), the support structure including: an outer structure (210) comprising an outer face (216) configured for engaging the interior face of a capsular bag of an eye, the outer face (216) having an equatorial region (222) and including an anterior region (220) and a posterior region (224) disposed on opposite sides of the equatorial region (222); an intermediate structure (212) operably coupled to the outer structure (210) and the optic body (21, 52, 202); an adhesive material disposed over at least a portion of the posterior region (224), **characterized in that** the adhesive material can be switched from a state that prevents cellular attachment to a state that promotes it; wherein, under a predetermined condition, the posterior region (224) has an adhesion that is greater than an adhesion of the anterior region (220).

2. The accommodating intraocular lens (50, 200) of claim 1, wherein the adhesive material is selected from the group consisting of a thermo-reversible bioadhesive polymer and a plurality of microfibers.

3. The accommodating intraocular lens (50, 200) of claim 1 or 2, wherein the intermediate structure (212) of the support structure (23, 204) includes a plurality of legs that extend radially outward from an edge of the optic body (21, 52, 202).

4. The accommodating intraocular lens (50, 200) of any one of claims 1 to 3, wherein the adhesive material is disposed over at least a portion of the equatorial region (222) of the outer face (216).

5. The accommodating intraocular lens (50, 200) of any one of claims 1 to 4, wherein the support structure (23, 204) is configured to change the shape of the optic body (21, 52, 202), translate the optic body (21, 52, 202) along the optical axis (OA), or both change the shape of the optic body (21, 52, 202) and translate the optic body (21, 52, 202) along the optical axis (OA).

6. The accommodating intraocular lens (50, 200) of any one of claims 1 to 5, wherein the adhesion of the posterior region (224) of the outer face (216) is an amount sufficient to maintain a constant contact area between the posterior region (224) of the outer face (216) and a face of a mating surface in contact with the outer face (216) when a force between the faces radially stretches an outer diameter of the support structure (23, 204) by 10 percent from a natural state of the accommodating intraocular lens (50, 200).

7. The accommodating intraocular lens (50, 200) of claim 1, wherein the adhesive material is a thermo-reversible bioadhesive polymer, the predetermined condition is a temperature that is greater than or equal to an average body temperature, and the adhesion of the posterior region (224) is an adhesion to an interior face of a human capsular bag.

8. The accommodating intraocular lens (50, 200) of claim 7, wherein the adhesive material has a first adherence value when at a temperature that is a predetermined amount below the average body temperature and a second adherence value when at a temperature that is at or above the average body temperature, the second adherence value being greater than the first adherence value.

9. The accommodating intraocular lens (50, 200) of claim 8, wherein either the predetermined amount is at least two degrees Celsius, or wherein the second adherence value is at least twice that of the first adherence value.

10. The accommodating intraocular lens (50, 200) of claim 7, wherein the adhesive material has a transition temperature that is at least two degrees Celsius less than the average body temperature.

11. The accommodating intraocular lens (50, 200) of claim 7, wherein the adhesion of the posterior region (224) of the outer face (216) is an amount sufficient to maintain a constant contact area between the posterior region (224) of the outer face (216) and the inner face of the capsular bag when a force between the faces radially stretches an outer diameter of the support structure (23, 204) by 10 percent from a natural state of the accommodating intraocular lens (50, 200).

12. The accommodating intraocular lens (50, 200) of claim 11, wherein the adhesion of the anterior region (220) of the outer face (216) is sufficiently low so that a contact area decreases between the anterior region (220) of the outer face (216) and the inner face of the capsular bag when the force between the faces radially stretches the outer diameter of the support structure (23, 204) by 10 percent from the natural state of the accommodating intraocular lens (50, 200).

13. The accommodating intraocular lens (50, 200) of claim 12, wherein the contact area decreases by at least 10 percent when the force between the faces radially stretches the outer diameter of the support structure (23, 204) by 10 percent from the natural state of the accommodating intraocular lens (50, 200).

## Patentansprüche

1. Anpassungsfähige Intraokularlinse (50, 200) zur Implantation in einen Kapselsack eines Auges, die umfasst:
einen einstellbaren optischen Körper (21, 52, 202), der um eine optische Achse (OA) herum angeordnet ist und eine vordere Fläche (205) und eine hintere Fläche (206) aufweist, wobei die Flächen (205, 206) eine freie Öffnung (207) begrenzen; eine Haltestruktur (23, 204), die so ausgelegt ist, dass sie eine Okularstärke von einem Kapselsack zu dem optischen Körper (21, 52, 202) überträgt, wobei die Haltestruktur aufweist: eine äußere Struktur (210), die eine äußere Fläche (216) umfasst, welche so ausgeführt ist, dass sie mit einer inneren Fläche eines Kapselsacks des Auges zusammengreift, wobei die äußere Fläche (216) eine Äquatorialregion (222) aufweist und eine vordere Region (220) und eine hintere Region (224) aufweist, die auf einander gegenüberliegenden Seiten der Äquatorialregion (222) angeordnet sind; eine Zwischenstruktur (212), die mit der äußeren Struktur (210) und dem optischen Körper (21, 52, 202) in Wirkverbindung steht; ein Haftvermittlermaterial, das zumindest über einem Abschnitt der hinteren Region (224) angeordnet ist,
**dadurch gekennzeichnet, dass**
das Haftvermittlermaterial von einem Zustand, in dem eine zelluläre Anhaftung verhindert wird, zu einem Zustand, in dem diese gefördert wird, umgeschaltet werden kann; wobei unter einer vorbestimmten Bedingung die hintere Region (224) eine Haftung aufweist, die größer ist als eine Haftung der vorderen Region (220).

2. Anpassungsfähige Intraokularlinse (50, 200) nach Anspruch 1, wobei das Haftvermittlermaterial aus der Gruppe bestehend aus einem thermoreversiblen bioadhäsiven Polymer und einer Vielzahl von Mikrofasern ausgewählt ist.

3. Anpassungsfähige Intraokularlinse (50, 200) nach Anspruch 1 oder 2, wobei die Zwischenstruktur (212) der Haltestruktur (23, 204) eine Vielzahl von Beinen aufweist, die sich von einem Rand des optischen Körpers (21, 52, 202) radial nach außen erstrecken.

4. Anpassungsfähige Intraokularlinse (50, 200) nach einem der Ansprüche 1 bis 3, wobei das Haftvermittlermaterial zumindest über einem Abschnitt der Äquatorialregion (222) der äußeren Fläche (216) angeordnet ist.

5. Anpassungsfähige Intraokularlinse (50, 200) nach einem der Ansprüche 1 bis 4, wobei die Haltestruktur (23, 204) so ausgeführt ist, dass sie die Form des optischen Körpers (21, 52, 202) verändert, den optischen Körper (21, 52, 202) entlang der optischen Achse (OA) umsetzt oder sowohl die Form des optischen Körpers (21, 52, 202) verändert als auch den optischen Körper (21, 52, 202) entlang der optischen Achse (OA) umsetzt.

6. Anpassungsfähige Intraokularlinse (50, 200) nach einem der Ansprüche 1 bis 5, wobei die Haftung der hinteren Region (224) der äußeren Fläche (216) ausreicht, um einen konstanten Kontaktbereich zwischen der hinteren Region (224) der äußeren Fläche (216) und einer Fläche einer Passfläche, die mit der äußeren Fläche (216) in Kontakt steht, aufrechtzuerhalten, wenn eine Kraft zwischen den Flächen einen Außendurchmesser der Haltestruktur (23, 204) um 10 Prozent aus einem natürlichen Zustand der anpassungsfähigen Intraokularlinse (50, 200) radial dehnt.

7. Anpassungsfähige Intraokularlinse (50, 200) nach Anspruch 1, wobei das Haftvermittlermaterial ein thermoreversibles bioadhäsives Polymer ist, die vorbestimmte Bedingung eine Temperatur ist, welche größer als eine oder gleich einer mittlere(n) Körpertemperatur ist, und die Haftung der hinteren Region (224) eine Haftung an einer inneren Fläche eines menschlichen Kapselsacks ist.

8. Anpassungsfähige Intraokularlinse (50, 200) nach Anspruch 7, wobei das Haftvermittlermaterial einen ersten Haftfähigkeitswert aufweist bei einer Temperatur, die um einen vorbestimmten Betrag unter der mittleren Körpertemperatur liegt, und einen zweiten Haftfähigkeitswert aufweist bei einer Temperatur, die bei oder über der mittleren Körpertemperatur liegt, wobei der zweite Haftfähigkeitswert größer ist als der erste Haftfähigkeitswert.

9. Anpassungsfähige Intraokularlinse (50, 200) nach Anspruch 8, wobei entweder der vorbestimmte Betrag mindestens zwei Grad Celsius beträgt oder wobei der zweite Haftfähigkeitswert mindestens zweimal der erste Haftfähigkeitswert ist.

10. Anpassungsfähige Intraokularlinse (50, 200) nach Anspruch 7, wobei das Haftvermittlermaterial eine Übergangstemperatur aufweist, die mindestens zwei Grad Celsius niedriger ist als die mittlere Körpertemperatur.

11. Anpassungsfähige Intraokularlinse (50, 200) nach Anspruch 7, wobei die Haftung der hinteren Region (224) der äußeren Fläche (216) ausreicht, um einen konstanten Kontaktbereich zwischen der hinteren Region (224) der äußeren Fläche (216) und der inneren Fläche des Kapselsacks aufrechtzuerhalten, wenn eine Kraft zwischen den Flächen einen Außendurchmesser der Haltestruktur (23, 204) um 10 Prozent aus einem natürlichen Zustand der anpassungsfähigen Intraokularlinse (50, 200) radial dehnt.

12. Anpassungsfähige Intraokularlinse (50, 200) nach Anspruch 11, wobei die Haftung der vorderen Region (220) der äußeren Fläche (216) ausreichend niedrig ist, so dass sich ein Kontaktbereich zwischen der vorderen Region (220) der äußeren Fläche (216) und der inneren Fläche des Kapselsacks verkleinert, wenn die Kraft zwischen den Flächen den Außendurchmesser der Haltestruktur (23, 204) um 10 Prozent aus dem natürlichen Zustand der anpassungsfähigen Intraokularlinse (50, 200) radial dehnt.

13. Anpassungsfähige Intraokularlinse (50, 200) nach Anspruch 12, wobei sich der Kontaktbereich um mindestens 10 Prozent verkleinert, wenn die Kraft zwischen den Flächen den Außendurchmesser der Haltestruktur (23, 204) um 10 Prozent aus dem natürlichen Zustand der anpassungsfähigen Intraokularlinse (50, 200) radial dehnt.

## Revendications

1. Lentille intraoculaire accommodative (50, 200) pour implantation dans un sac capsulaire d'un oeil, comprenant : un corps optique ajustable (21, 52, 202) disposé autour d'un axe optique (OA) et comportant une face antérieure (205) et une face postérieure (206), les faces (205, 206) définissant une ouverture transparente (207) ; une structure de support (23, 204) adaptée pour transférer une force oculaire d'un sac capsulaire au corps optique (21, 52, 202), la structure de support comportant : une structure extérieure (210) comprenant une face extérieure (216) configurée pour engager la face intérieure d'un sac capsulaire d'un oeil, la face extérieure (216) ayant une région équatoriale (222) et comportant une région antérieure (220) et une région postérieure (224) disposées sur des côtés opposés de la région équatoriale (222) ; une structure intermédiaire (212) fonctionnellement couplée à la structure extérieure (210) et au corps optique (21, 52, 202) ; un matériau adhésif disposé sur au moins une partie de la région postérieure (224), **caractérisée en ce que** le matériau adhésif peut être basculé d'un état qui empêche la fixation cellulaire à un état qui la favorise ; dans laquelle, dans une condition prédéterminée, la région postérieure (224) a une adhérence qui est supérieure à une adhérence de la région antérieure (220).

2. Lentille intraoculaire accommodative (50, 200) de la revendication 1, dans laquelle le matériau adhésif est choisi dans le groupe constitué par un polymère bioadhésif thermoréversible et une pluralité de microfibres.

3. Lentille intraoculaire accommodative (50, 200) de la revendication 1 ou 2, dans laquelle la structure intermédiaire (212) de la structure de support (23, 204) comporte une pluralité de pattes qui s'étendent radialement vers l'extérieur depuis un bord du corps optique (21, 52, 202).

4. Lentille intraoculaire accommodative (50, 200) de l'une quelconque des revendications 1 à 3, dans laquelle le matériau adhésif est disposé sur au moins une partie de la région équatoriale (222) de la face extérieure (216).

5. Lentille intraoculaire accommodative (50, 200) de l'une quelconque des revendications 1 à 4, dans laquelle la structure de support (23, 204) est configurée pour modifier la forme du corps optique (21, 52, 202), translater le corps optique (21, 52, 202) le long de l'axe optique (OA), ou à la fois modifier la forme du corps optique (21, 52, 202) et translater le corps optique (21, 52, 202) le long de l'axe optique (OA).

6. Lentille intraoculaire accommodative (50, 200) de l'une quelconque des revendications 1 à 5, dans laquelle l'adhérence de la région postérieure (224) de la face extérieure (216) est une quantité suffisante pour maintenir une zone de contact constant entre la région postérieure (224) de la face extérieure (216) et une face d'une surface d'accouplement en contact avec la face extérieure (216) quand une force entre les faces étire radialement un diamètre extérieur de la structure de support (23, 204) de 10 pour cent à partir d'un état naturel de la lentille intraoculaire accommodative (50, 200).

7. Lentille intraoculaire accommodative (50, 200) de la revendication 1, dans laquelle le matériau adhésif est un polymère bioadhésif thermoréversible, la condition prédéterminée est une température qui est supérieure ou égale à une température corporelle moyenne, et l'adhérence de la région postérieure (224) est une adhérence sur une face intérieure d'un sac capsulaire humain.

8. Lentille intraoculaire accommodative (50, 200) de la revendication 7, dans laquelle le matériau adhésif a une première valeur d'adhérence quand il se trouve à une température qui est inférieure d'une quantité prédéterminée à la température corporelle moyenne et une deuxième valeur d'adhérence quand il se trouve à une température qui est égale ou supérieure à la température corporelle moyenne, la deuxième valeur d'adhérence étant supérieure à la première valeur d'adhérence.

9. Lentille intraoculaire accommodative (50, 200) de la revendication 8, dans laquelle la quantité prédéterminée est d'au moins deux degrés Celsius, ou dans laquelle la deuxième valeur d'adhérence est égale à au moins deux fois la première valeur d'adhérence.

10. Lentille intraoculaire accommodative (50, 200) de la revendication 7, dans laquelle le matériau adhésif a une température de transition qui est inférieure d'au moins deux degrés Celsius à la température corporelle moyenne.

11. Lentille intraoculaire accommodative (50, 200) de la revendication 7, dans laquelle l'adhérence de la région postérieure (224) de la face extérieure (216) est une quantité suffisante pour maintenir une zone de contact constant entre la région postérieure (224) de la face extérieure (216) et la face intérieure du sac capsulaire quand une force entre les faces étire radialement un diamètre extérieur de la structure de support (23, 204) de 10 pour cent à partir d'un état naturel de la lentille intraoculaire accommodative (50, 200).

12. Lentille intraoculaire accommodative (50, 200) de la revendication 11, dans laquelle l'adhérence de la région antérieure (220) de la face extérieure (216) est suffisamment faible pour qu'une zone de contact entre la région antérieure (220) de la face extérieure (216) et la face intérieure du sac capsulaire diminue quand la force entre les faces étire radialement le diamètre extérieur de la structure de support (23, 204) de 10 pour cent à partir de l'état naturel de la lentille intraoculaire accommodative (50, 200).

13. Lentille intraoculaire accommodative (50, 200) de la revendication 12, dans laquelle la zone de contact diminue d'au moins 10 pour cent quand la force entre les faces étire radialement le diamètre extérieur de la structure de support (23, 204) de 10 pour cent à partir de l'état naturel de la lentille intraoculaire accommodative (50, 200).
